# EUROPÄISCHE PATENTSCHRIFT

(11) **EP 0 373 733 B1**
(45) Veröffentlichungstag und Bekanntmachung des Hinweises auf die Patenterteilung: **22.09.1993**
(21) Anmeldenummer: 89250103.2
(22) Anmeldetag: 04.12.1989
(51) Int. Cl.: A61F 2/08, A61B 17/56

(54) **Implantierbares Befestigungsmittel für extra-orale Applikationen**
Implantable fixation means for extra-oral applications
Moyens de fixation implantables pour applications extra-orales

(30) Priorität: 10.12.1988 DE 3841704
(43) Veröffentlichungstag der Anmeldung: 20.06.1990
(73) Patentinhaber: IMZ-Fertigungs- und Vertriebsgesellschaft für dentale Technologie mbH, 70794 Filderstadt (DE)
(72) Erfinder: Kirsch, Axel, Dr., D-7024 Filderstadt (DE)
(74) Vertreter: Goddar, Heinz J., Dr.

(56) Entgegenhaltungen:
- EP-A- 0 282 789
- WO-A-86/03666
- GB-A- 2 162 753
- US-A- 3 988 783
- US-A- 4 781 591

## Beschreibung

Die vorliegende Erfindung betrifft ein implantierbares Befestigungsmittel für extra-orale Applikation, mit einem in den Knochen implantierbaren Grundkörper mit einer gewindepressenden oder -schneidenden Wendel nahe dem oberen Rand des Grundkörpers und einem mit dem Grundkörper verbindbaren Implantatpfosten.

Seit vielen Jahren schon wird die Implantation von künstlichen Ligamenten, die gegeneinander bewegliche Teile des menschlichen Skeletts verbinden, praktiziert. Wenn beispielsweise bei Schädigungen oder chronischer Instabilität der Kreuzbänder des Kniegelenks eine intraartikuläre autologe Rekonstruktion versagt, kann ein Kreuzbandersatz aus synthetischem Material implantiert werden. Hierfür hat sich beispielsweise ein besonderes Polytetrafluorethylen, das unter der Bezeichnung GORE-TEX® bekannt geworden ist, bewährt, da dieses biochemisch inert und deshalb besonders körperverträglich ist.

Lange Zeit ist man davon ausgegangen, daß die Befestigungsmittel für derartige künstliche Ligamente nur eine untergeordnete Rolle spielen, da diese selbst im Laufe der Zeit mit körpereigenem Gewebe zusammenwachsen. Vor einiger Zeit wurde jedoch festgestellt, daß die Gewebereaktion auf einen implantierten Kreuzbandersatz sehr unterschiedlich ist, so daß nicht in jedem Fall von einem verläßlichen Einwachsen des künstlichen Ligaments in das körpereigene Gewebe ausgegangen werden kann (Rushton N.,Dandy DJ, Naylor CPE. The clinical, arthroscopic and histological findings after replacement of the anterior cruciate ligament with carbon-fibre. J Bone Joint Surg (Br) 1983: 65-B: 308-9.) In vielen Fällen also müssen die Befestigungsmittel über einen langen Zeitraum ihre Funktion erfüllen, um beispielsweise den großen Kräften widerstehen zu können, die bereits bei normalen Bewegungsabläufen im Kniegelenk auftreten.

Zur Befestigung von künstlichen Ligamenten gibt es in Literatur und Praxis bereits mehrere Vorschläge, Neben der herkömmlichen Sherman-Knochenschraube wurden spezielle Kortikalis-Schrauben für bestimmte Fixationsösen entwickelt. Weiterhin kamen sogenannte Richards-Klammern zum Einsatz, die für einen sicheren Sitz im vorgebohrten Knochen Widerhaken aufweisen. Pollerartige Befestigungsmittel aus einem Kohlenstofffaser/Polysulfon-Werkstoff weisen einen gespaltenen Schaft auf, der durch Einschlagen eines Mittelstifts gespreizt wird. Schließlich wurden noch spezielle implantierbare Befestigungsknebel und im Zusammenhang mit Kortikalis-Schrauben besondere Durchgangshülsen vorgeschlagen (Andrew A. Amis, The strength of artificial ligament anchorages, J. Bone Joint Surg (Br) 1988: 70-B: 397-403).

Die bisher bekannten Befestigungsmittel für künstliche Ligamente sind entweder sehr aufwendig hinsichtlich ihrer Implantation oder nicht stabil genug, um die ungewöhnlich großen Kräfte, die beispielsweise im Bereich des Kniegelenks auftreten, auf Dauer ohne Beschädigungen zu überstehen. Daher kommt es immer wieder zu Verformungen oder sogar Brüchen der Befestigungsmittel oder Schädigungen des umgebenden Knochenmaterials, was in jedem Fall erneut aufwendige operative Eingriffe erforderlich macht.

Befestigungsmittel für extra-orale Applikationen werden aber nicht nur für künstliche Ligamente, sondern auch in anderen Bereichen benötigt.

Bei schweren Hautverletzungen, wie z. B. starken Verbrennungen, ist es, insbesondere zur Vermeidung von Infektionen, notwendig, die betroffenen Partien möglichst umgehend mit dafür geeignetem Material abzudecken. Dabei ergeben sich insbesondere Probleme im Hinblick auf eine schnelle und sichere Befestigung des Abdeckmaterials, da die zerstörten Bereiche besonders druck- und schmerzempfindlich sind und gleichzeitig z. B. im Gesichtsbereich eine ausgeprägte Plastizität und Kleinflächigkeit aufweisen können. Zur Lösung dieser Probleme ist bisher noch kein praktikabler Vorschlag bekannt geworden.

Ein Befestigungsmittel der eingangs genannten Gattung ist aus der US-A 4,781,591 oder der GB-A 2 162 753 bekannt.

Dabei zeigt die US-A 4,781,591, ebenso wie die GB-A 2 162 753, einen Gewindekörper, bei dem die gewindepressende oder gewindeschneidende Wendel sich im wesentlichen über die gesamte Länge des Grundkörpers erstreckt. Für den Operateur ist es verhältnismäßig schwierig, mittels dieser bekannten Befestigungsmittel unter Schneiden des notwendigen Gewindes in der vorher in den Knochen eingebrachten Bohrung ein schnelles, präzises Anziehen des Befestigungsmittels zu gewährleisten.

Während in der US-A 4,781,591 keinerlei besondere Einrichtungen zum Versenken des Befestigungsmittels in dem Knochen angegeben sind, schlägt die GB-A 2 162 753 vor, das obere Ende des Grundkörpers mit einander gegenüberliegenden Einkerbungen zu versehen, in die ein entsprechend ausgebildeter Schraubenzieher eingreifen kann. Ein optimales Ausrichten des Grundkörpers insbesondere auch im Hinblick darauf, daß gegebenenfalls ein Implantatpfosten aufzunehmen ist, ist mit den bekannten Befestigungsmitteln unmöglich.

Der Erfindung liegt daher die Aufgabe zugrunde, das gattungsgemäße implantierbare Befestigungsmittel dahingehend weiterzubilden, daß es schnell und zuverlässig in jedes nur denkbare Knochenmaterial implantiert werden kann, wobei für daran anzubringende Implantate gewährleistet sein soll, daß sie präzise ausgerichtet werden können.

Erfindungsgemäß wird diese Aufgabe durch ein implantierbares Befestigungsmittel nach Patentanspruch 1 oder Patentanspruch 2 gelöst. Vorteilhafte Ausgestaltungen sind Gegenstand der Ansprüche 3 bis 6.

Der Erfindung liegt einerseits die überraschende Erkenntnis zugrunde, daß es gelingt, durch die erfindungsgemäße Ausbildung der Wendel, welche nur nahe dem oberen Rand des Grundkörpers vorgesehen ist, in Verbindung mit dem in die Blindbohrungen an der Stirnfläche des Grundkörpers einsetzbaren Spezialkörper ein einwandfreies und schnelles, dabei sehr präzises Anziehen des Grundkörpers im Knochen unter gut zentriertem Schneiden des notwendigen Gewindes zu bewirken, wobei der nicht mit der Wendel versehene Teil des Grundkörpers als Zentrierhilfe wirkt. Damit wird beispielsweise auch die genaue Ausrichtung eines Implantates gewährleistet.

Nach einem anderen Aspekt der Erfindung ist vorgesehen, daß der Kopf des Implantatpfostens mit einer mangetischen oder nicht magnetischen Haftfläche versehen ist. Bei Verwendung als Befestigungsmittel für Abdeckmaterial für Verletzungen kann der erfindungsgemäße Grundkörper insbesondere im gesamten Kopfbereich besonders einfach implantiert werden, da hier an besonders vielen Stellen Knochen direkt unter der Haut zu finden ist, so daß bei einer Implantation kaum Muskelgewebe zerstört werden muß. Das Befestigungsmittel kann schnell und zuverlässig rund um die zerstörten Bereiche angebracht werden und dann zur Befestigung des notwendigen Abdeckmaterials dienen. Das erfindungsgemäße Befestigungsmittel ist selbstverständlich nicht nur auf die bereits erwähnten Anwendungsfälle beschränkt, sondern kann zur entsprechenden Befestigung beliebiger Materialien an Knochen verwendet werden. So ist beispielsweise auch eine Fixierung von Haarersatzteilen auf diese Weise möglich. Da bei der Befestigung des Abdeckmaterials keine großen Zugkräfte auftreten ist erfindungsgemäß vorgesehen, daß in den Kopf des Implantatpfostens ein Magnet eingelassen ist oder daß Klettverschlüsse, Klebeflächen oder andere äquivalente Befestigungsmittel vorgesehen sind.

Dabei schlägt die Erfindung vor, daß sich die Wendel über maximal 1,5, besonders bevorzugt über 1 bis 1,25 Umdrehungen des Grundkörpers erstreckt und ggf. eine Steigung von zwischen 1 und 2, besonders bevorzugt von 1,5, aufweist. Bei einer derartigen Ausgestaltung der Wendel ist es möglich, den Grundkörper sicher im Knochenmaterial zu verankern, ohne ein Gewinde über dessen ganze Länge vorsehen zu müssen. Bei Verwendung als Befestigungsmittel für Abdeckmaterial von Verletzungen kann der Grundkörper sogar kurz unterhalb der Wendel enden, d. h. relativ kurz gehalten und daher besonders schnell und doch gleichzeitig zuverlässig in das Knochenmaterial eingedreht werden. Damit wird gerade bei diesem Anwendungsfall ein sicherer Sitz des Grundkörpers im Knochenmaterial ermöglicht, ohne daß dieser zu tief eindringen muß.

Die Wendel selbst kann mit einem Flankenwinkel von etwa 60° oder bei Einsatz in besonders empfindlichen Knochenbereichen, wie z. B. im Kopfbereich, bei denen eine scharfkantige Wendel leicht zu Rissen oder Absplitterungen führen könnte, rund ausgebildet werden.

Als besonders günstiges Material für den Grundkörper hat sich aufgrund seiner geringen Anfälligkeit gegenüber Körperflüssigkeiten sowie seiner hohen Stabilität gegenüber Zugkräften Titan oder eine Titanlegierung erwiesen.

Es kann dabei der Grundkörper von außen gewebefreundlich beschichtet sein. Am geeignesten hat sich dazu eine Plasmabeschichtung aus Hydroxylapatit erwiesen. Mit diesem Material, das unter anderem auch Hauptbestandteil des Zahnschmelzes ist, können zuverlässig Abstoßungsreaktionen des den Grundkörper umgebenden Gewebes vermieden werden.

Um ein unbeabsichtigt zu tiefes Eindringen des Grundkörpers in das Knochenmaterial zu verhindern, was insbesondere bei empfindlichen Knochenbereichen wichtig ist, sollte der Grundkörper einen verbreiterten oberen Randbereich aufweisen.

Als vorteilhafte Ausführungsform der Erfindung hat sich weiterhin erwiesen, eine Distanzhülse vorzusehen, die als Verlängerung auf den oberen Rand des Grundkörpers aufsetzbar ist. Ein besonderer Vorteil der Distanzhülse besteht dabei darin, daß diese den Grundkörper der zunächst unter der sich wieder schließenden Haut in den Knochen einheilt, starr bis über deren Oberkante verlängert werden kann, ohne daß diese durch Verformungsbewegungen gereizt wird. Vorteilhafterweise besteht die Distanzhülse aus elektrisch isolierendem Material. Hierfür bietet sich insbesondere keramische Material, wie z. B. Aluminiumoxid, Magnesiumoxid und/oder Zirkonoxid an, um eine möglichst gute Hautverträglichkeit der Distanzhülse zu gewährleisten. Als vorteilhaft hat sich bei der Ausgestaltung der Distanzhülse weiterhin erwiesen, daß diese Mittels eines Innenkonus in den Grundkörper eingreift, damit eine kräftige reib- und formschlüssige Verbindung der Teile sichergestellt ist.

So können im zu befestigenden Material Löcher oder Lochreihen vorgesehen werden, in die der Implantatpfosten eingreift. In einer Ausführungsform der Erfindung weist der Implantatpfosten einen verbreiterten Kopf auf, der nach Durchstecken des Implantatpfostens durch eines der Befestigungslöcher das zu befestigende Material übergreift und fixiert. Diese Fixierung kann aber in einer anderen Ausführungsform der Erfindung auch mittels eines oder mehrerer Haken am Kopf des Implantatpfosten bewerkstelligt werden. Bei der Befestigung von künstlichen Ligamenten kann das entsprechende Faserbündel beispielsweise auch zu rechteckförmigen Ansatzstücken verschmolzen werden, in die entsprechend geformte Implantatpfosten bereits eingeschmolzen werden. Um eine entsprechen de Längenanpassung des künstlichen Ligaments vornehmen zu können ist es auch vorteilhaft, an dessen Enden mehrere Lochreihen vorzusehen.

Weitere Einzelheiten und Vorteile der Erfindung werden nachstehend anhand der Zeichnungen noch näher erläutert.

Dabei zeigen
- Fig. 1: einen Längsschnitt durch eine bevorzugte Ausführungs form des erfindungsgemäßen Befestigungsmittels zur Verwendung bei künstlichen Ligamenten;
- Fig. 2: einen Längsschnitt durch eine andere Ausführungsform des erfindungsgemäßen Befestigungsmittels, ebenfalls zur Verwendung bei künstlichen Ligamenten, aber um eine Distanzhülse verlängert;
- Fig. 3: einen Längsschnitt durch eine weitere Ausführungsform des erfindungsgemäßen Befestigungsmittels zur Verwendung für Abdeckmaterial von Verletzungen; und
- Fig. 4: eine Draufsicht auf die Ausführungsform nach Fig. 3.

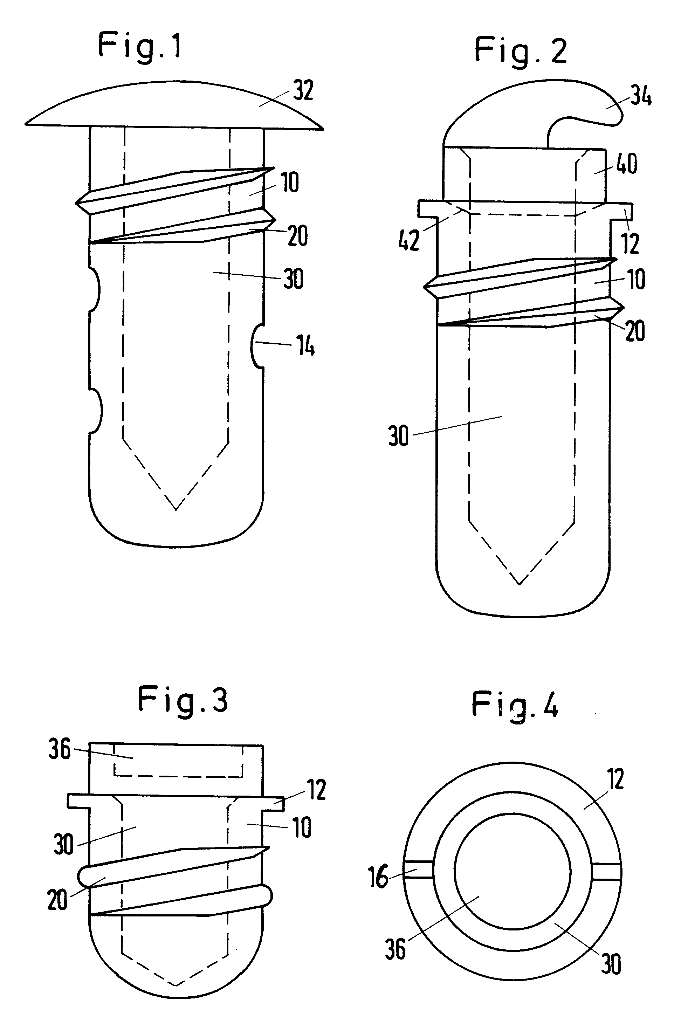

Im einzelnen zeigt Fig. 1 ein erfindungsgemäßes Befestigungsmittel mit einem Grundkörper 10, der in eine Vorbohrung einer freigelegten Knochenpartie des Patienten eingedreht wird und dort ggf. knöchern einheilen kann. Der Grundkörper 10 besteht vorzugsweise aus Titan oder einer Titanlegierung und ist auf seiner Außenfläche entweder durch Rändeln oder Sandstrahlen aufgerauht oder in der bereits oben geschilderten Art und Weise gewebefreundlich beschichtet. Um das knöcherne Einwachsen des Grundkörpers zu begünstigen können Vertiefungen 14, sogenannten Lakonen, oder Durchbrüche im Grundkörper vorgesehen sein. Dies hat insbesondere bei der Verwendung des erfindungsgemäßen Befestigungsmittels für künstliche Ligamente Bedeutung.

Der Grundkörper 10 ist nach oben offen und an seiner Innenfläche so ausgebildet, daß der Implantatpfosten 30 eingeschraubt oder bajonettartig einsteckt werden kann. Der Kopf 32 des Implantatpfostens ist dabei pilzartig verbreitert, um nach Durchstecken des Implantatpfostens durch entsprechend vorgesehene Löcher im zu befestigenden Material, wie z. B. Fixationsösen oder -löcher, in Eingriff mit diesen zu kommen.

Direkt unter der Stirnfläche des Grundkörpers 10 ist eine Wendel mit einem Gewindegang von 1,25, einer Steigung von 1,5 und einem Flankenwinkel von 60° vorgesehen, die gewindeschneidend in das Knochenmaterial eindringt. Bei dem Ausführungsbeispiel gemäß Fig. 2 ist gegenüber Fig. 1 eine Distanzhülse 40 vorgesehen, die mittels eines Innenkonus 42 in den Grundkörper 10 eingreift. Darüber hinaus ist in diesem Fall der Kopfteil des Implantatpfostens 30 in Form eines Hakens 34 ausgebildet, der ebenso wie die pilzförmige Verbreiterung 32 in Fig. 1 zum Eingriff mit entsprechenden Fixationsösen oder -löchern des zu befestigenden Materials kommen soll.

Fig. 3 schließlich zeigt eine Ausführungsform des erfindungsgemäßen Befestigungsmittels, wie sie bevorzugt zur Befestigung von Abdeckmaterial für Verletzungen verwendet werden kann. Der Kopf 12 des Grundkörpers 10 ist dabei verbreitert, um ein versehentlich zu tiefes Eindrehen des Grundkörpers 10 in den Knochen zu verhindern. Insgesamt ist der Grundkörper 10 sehr kurz ausgebildet, da er bereits direkt unter der Wendel 20 endet. Die Wendel 20 selbst, die ebenfalls einen Gewindegang von 1,25 und eine Steigung von 1,5 aufweist ist,in diesem Fall rund ausgebildet und drückt sich daher gewindepressend in das Knochenmaterial. Am Kopf des Implantatpfostens ist ein Magnet 36 vorgesehen, der in Wechselwirkung mit einem entsprechenden Gegenmagneten zur Befestigung von beispielsweise Abdeckmaterial für Verletzungen oder aber auch verschiedenstartiger anderer Materialien, wie z. B. Haarteilersatz, dienen kann.

Fig. 4 schließlich zeigt eine Draufsicht auf die Ausführungsform des erfindungsgemäßen Befestigungsmittels nach Fig. 3. Am Rande des verbreiterten Kopfs 12 des Grundkörpers 10 erkennt man einander gegenüberliegenden Blindbohrungen 16 zum Eingriff eines für einen schnellen Ein- und Ausbau des Grundkörpers benötigten Spezialschlüssels. Der mittlere Kreis in dieser Darstellung umschreibt den Umfang des Implantatpfostenkopfes, der in seiner Mitte eine runde Magnetplatte 36 trägt. An dessen Stelle können selbstverständlich auch die bereits weiter oben erwähnten anderen Befestigungsmöglichkeiten, wie z. B. eine Haftfläche für einen Klettverschluß oder eine Klebefläche, vorgesehen sein.

## Patentansprüche

1. Implantierbares Befestigungsmittel für extra-orale Applikationen, mit einem in den Knochen implantierbaren Grundkörper (10) mit einer gewindepressenden oder -schneidenden Wendel (20) nahe dem oberen Rand des Grundkörpers und einem mit dem Grundkörper verbindbaren Implantatpfosten, dadurch gekennzeichnet, daß der Kopf des Implantatpfostens (30) Pilzförmig (32) verbreitert oder in Form eines Hakens (34) ausgebildet ist; daß die Wendel (20) sich über maximal 1,5 Umdrehungen des Grundkörpers (10) erstreckt; daß die Wendel (20) eine Steigung zwischen 1 und 2 aufweist; und daß der Grundkörper (10) an seiner oberen Stirnfläche Blindbohrungen (16) zum Ansetzen eines Spezialschlüssels aufweist.

2. Implantierbares Befestigungsmittel für extra-orale Applikation, mit einem in den Knochen inplantierbaren Grundkörper mit einer gewindepressenden oder -schneidenden Wendel nahe dem oberen Rand des Grundkörpers und einem mit dem Grundkörper verbindbaren Implantatpfosten, dadurch gekennzeichnet, daß in den Kopf des Implantatpfostens (30) ein Magnet (36) eingelassen ist oder der Kopf des Implantatpfostens (30) mindestens eine Haftfläche für einen Klettverschluß oder mindestens eine Klebefläche aufweist; daß die Wendel (20) sich über maximal 1,5 Umdrehungen des Grundkörpers (10) erstreckt; und daß der Grundkörper (10) kurz unterhalb der Wendel (20) endet.

3. Befestigungsmittel nach Anspruch 1 oder 2, dadurch gekennzeichnet, daß die Wendel (20) sich über 1 bis 1,25 Umdrehungen des Grundkörpers (10) erstreckt.

4. Befestigungsmittel nach einem der Ansprüche 1 bis 3, dadurch gekennzeichnet, daß eine Distanzhülse (40) vorgesehen ist, die als Verlängerung auf den oberen Rand (12) des Grundkörpers (10) aufsetzbar ist.

5. Befestigungsmittel nach Anspruch 4, dadurch gekennzeichnet, daß die Distanzhülse (40) mit einem Innenkonus (42) in das offene Ende des Grundkörpers (10) eingreift.

6. Befestigungsmittel nach Anspruch 2, dadurch gekennzeichnet, daß die Wendel (20) rund ausgebildet ist.

## Claims

1. An implantable fixing means for extra-oral applications, comprising a basic member (10) adapted to be implanted in the bone and having a screwthread-pressing or screwthread-cutting helix (20) near the top edge of the member and on implant post connectable to the member, characterised in that the head of the implant post (30) is widened mushroom-fashion (32) or is constructed in the form of a hook (34); in that the helix (20) extends over no more than 1.5 turns of the basic member (10); in that the helix (20) has a pitch or between 1 and 2; and in that the base member (10) has blind bores (16) at the top end face for engagement with a special key.

2. An implantable fixing means for extra-oral applications, comprising a basic member adapted to be implanted in the bone and having a screwthread-pressing or screwthread-cutting helix near the top edge of the member and an implant post connectable to the member, characterised in that a magnet (36) is recessed into the head of the implant post (30) or the head of the implant post (30) has at least one adhesive surface for a hook and loop fastener or at least one adhesion surface; in that the helix (20) extends over no more than 1.5 turns of the basic member (10); and in that the basic member (10) terminates just below the helix (20).

3. A fixing means according to claim 1 or 2, characterised in that the helix (20) extends over 1 to 1.25 turns of the basic member (10).

4. A fixing means according to any one of claims 1 to 3, characterised in that a spacer bush (40) is provided and can be fitted as an extension to the top edge (12) of the basic member (10).

5. A fixing means according to claim 4, characterised in that the spacer bush (40) engages in the open end of the basic member (10) by an internal taper (42).

6. A fixing means according to claim 2, characterised in that the helix (20) is of round construction.

## Revendications

1. Moyen de fixation implantable pour applications extra-orales, comprenant un corps de base (10) qui peut être implanté dans l'os et qui comporte, à proximité de son bord supérieur, une hélice (20) de filetage par pression ou par taillage, et comprenant une tige d'implant qui peut être reliée au corps de base, caractérisé en ce que la tête de la tige d'implant (30) est élargie en forme de champignon (32) ou conçue en forme de crochet (34), en ce que l'hélice (20) s'étend au maximum sur 1,5 tour du corps de base (10), en ce que l'hélice (20) présente une pente comprise entre 1 et 2, et en ce que le corps de base (10) comporte, sur sa surface frontale supérieure, des trous borgnes (16) destinés à recevoir une clé spéciale.

2. Moyen de fixation implantable pour applications extra-orales, comprenant un corps de base qui peut être implanté dans l'os et qui comporte, à proximité de son bord supérieur, une hélice de filetage par pression ou par taillage, et comprenant une tige d'implant qui peut être reliée au corps de base, caractérisé en ce que, dans la tête de la tige d'implant (30) est logé un aimant (36) ou la tête de la tige d'implant (30) comporte au moins une surface adhérente pour une fermeture auto-agrippante ou au moins une surface adhésive, en ce que l'hélice (20) s'étend au maximum sur 1,5 tour du corps de base (10), et en ce que le corps de base (10) se termine juste en dessous de l'hélice (20).

3. Moyen de fixation selon la revendication 1 ou 2, caractérisé en ce que l'hélice (20) s'étend sur 1 à 1,25 tour du corps de base (10).

4. Moyen de fixation selon une des revendications 1 à 3, caractérisé en ce qu'il est prévu une douille d'écartement (40) qui peut s'adapter, en forme d'extension, sur le bord supérieur (12) du corps de base (10).

5. Moyen de fixation selon la revendication 4, caractérisé en ce qu'un cône interne (42) de la douille d'écartement (40) s'engage dans l'extrémité ouverte du corps de base (10).

6. Moyen de fixation selon la revendication 2, caractérisé en ce que l'hélice (20) est de forme ronde.
